(19) Europäisches Patentamt
European Patent Office
Office européen des brevets

(11) Publication number: 0 134 696
A1

(12) # EUROPEAN PATENT APPLICATION

(21) Application number: 84305247.3

(22) Date of filing: 02.08.84

(51) Int. Cl.[4]: C 07 D 231/08
C 07 D 231/54
//G03C5/54

(30) Priority: 04.08.83 US 520283

(43) Date of publication of application:
20.03.85 Bulletin 85/12

(84) Designated Contracting States:
DE FR GB NL

(71) Applicant: EASTMAN KODAK COMPANY
343 State Street
Rochester New York 14650(US)

(72) Inventor: Michno, Drake M.
687 Regina Drive
Webster New York 14580(US)

(74) Representative: Pepper, John Herbert et al,
KODAK LIMITED Patent Department P.O. Box 114 190
High Holborn
London WC1V 7EA(GB)

(54) **Preparation of 4-substituted-3-pyrazolidinones.**

(57) A process for the preparation of 4-substituted-3-pyrazolidinones comprising reacting a 3-pyrazolidinone or 2-pyrazoline compound, blocked with a removable blocking group, with an electrophilic moiety at a temperature of equal to or less than 0°C in the presence of a strong base. The electrophilic moiety selectively adds to the 4-position of the compound, and the blocking group is thereafter removed, The 4-substituted-3-pyrazolidinones are useful as electron transfer agents in diffusion transfer photography.

EP 0 134 696 A1

PREPARATION OF 4-SUBSTITUTED-3-PYRAZOLIDINONES

This invention relates to a process for preparing 4-substituted-3-pyrazolidinones which are useful as electron transfer agents in color diffusion transfer photography.

Many different methods for preparing 4-substituted-3-pyrazolidinones are known in the art. U.S. Patent 2,289,367 and European Patent 54,002 describe methods comprising reaction of a substituted hydrazine with a ß-halogenated or ß-hydroxy acid chloride or carboxylic acid. Alternatively, an acrylate ester is reacted with a substituted aromatic amine, then nitrosated and hydrolyzed to form the 3-pyrazolidinone.

A problem associated with these and other known methods is that they are impractical or impossible to use for preparing complex 4-substituted-3-pyrazolidinones. The use of ß-halogenated or ß-hydroxy acid chloride equivalents or acrylate starting materials require that the final 4-substituent of the pyrazolidinone be compatible with the harsh reaction conditions of the entire reaction sequence, so that relatively few 4-substituted-3-pyrazolidinones have previously been prepared. Further, acrylate starting materials cannot be directly used to prepare 4,4-disubstituted pyrazolidinones. A series of 4-substituted-3-pyrazolidinones cannot be prepared from a common intermediate by the prior art processes, so that each derivative requires its own long synthetic sequence. Further still, these known processes result in poor product yield. Thus, there is a need for a relatively direct, high yield process for the preparation of 4-substituted-3-pyrazolidinones.

The object of the present invention is to provide a process for the preparation of 4-substituted-3-pyrazolidinones which avoids the

disadvantages of the prior art processes discussed above. This object is achieved by the process of the invention, which process is characterized by

(1) reacting

A) a compound having the structural formula:

(i) 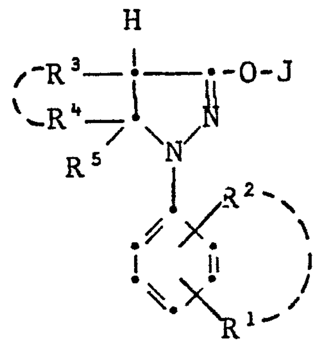 or

(ii) 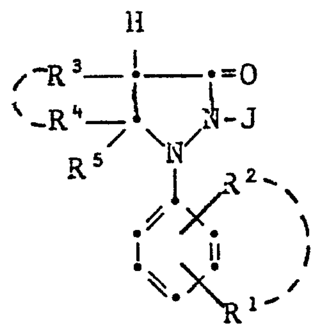

wherein:

$R^1$, $R^2$, $R^3$, $R^4$ and $R^5$ are independently hydrogen; fluorine; allyl; trimethylsilyl; benzyl; substituted or unsubstituted alkyl; alkoxy; or thioalkoxy; $R^1$ and $R^2$ may be taken together with carbon atoms in the phenyl ring to which they are attached to form a cyclic ring; or $R^3$ and $R^4$ may be taken together with the carbon atoms at the 4- and 5-positions of the pyrazolidinone ring to form a cyclic ring; and,

J is a removable blocking group;

the compound being stable at a temperature equal to or less than 0°C in the presence of a strong base having a pKa greater than 20; with

B) an electrophilic moiety at a temperature equal to or less than 0°C in the presence of a strong base having a pKa greater than 20, whereby said electrophilic moiety selectively adds to the C atom at the 4-position in the pyrazolidinone ring; and

(2) removing the blocking group.

A wide variety of 4-substituted-3-pyrazolidinones, including complex 4-substituted-3-pyrazolidinones and 4,4-disubstituted-3-pyrazolidinones, may be prepared by the process of the invention, many of which have been impractical or impossible to prepare by previously known methods. In addition, the final 4-substituent of the pyrazolidinone prepared by the process of the invention need only be compatible with the reaction conditions of the relatively direct process disclosed herein. Further, a series of 4-substituted-3-pyrazolidinones can be prepared from a single intermediate preformed pyrazolidinone and the relatively direct process of the present invention results in high yields of 4-substituted-3-pyrazolidinones.

As described above, $R^1$, $R^2$, $R^3$, $R^4$ and $R^5$ are independently hydrogen; fluorine; allyl; trimethylsilyl; benzyl; alkyl, preferably containing 1 to 7 carbon atoms such as methyl, ethyl, propyl or butyl, unsubstituted or substituted with groups such as hydroxyl; alkoxy, such as methoxy or ethoxy; or thioalkoxy, such as thiomethoxy or thioethoxy; $R^1$ and $R^2$ may be taken together with carbon atoms in the phenyl ring to which they are attached to form a cyclic ring, e.g., $R^1$ and $R^2$ may represent an alkylene group having from 1 to 4 carbon atoms such as methylene, ethylene, propylene or butylene, optionally

having 1 or more oxygen atoms in the group with or without additional unsaturation; or $R^3$ and $R^4$ may be taken together with the carbon atoms at the 4- and 5-positions of the pyrazolidinone ring to form a cyclic ring, e.g., $R^3$ and $R^4$ may represent an alkylene group having from 1 to 4 carbon atoms such as methylene, ethylene, propylene or butylene, optionally having 1 or more oxygen atoms in the group with or without additional unsaturation.

Preferred blocked intermediates, which are stable at a temperature of less than 0°C and in the presence of a strong base having a pKa greater than 20, are formed by two alternative embodiments of the invention. The first embodiment comprises reacting a 3-pyrazolidinone having the structural formula:

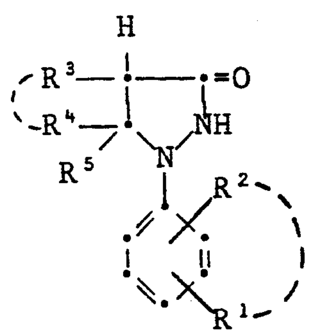

wherein:

$R^1$, $R^2$, $R^3$, $R^4$ and $R^5$ are defined as above, with a blocking group providing composition comprising t-butyl-dimethylsilyl chloride; benzyl bromide; or N,N-disubstituted carbamoyl chloride. The above blocking group providing compositions when reacted with a 3-pyrazolidinone in accordance with a preferred embodiment of the invention result in good yield of the O-blocked silyl and N,N-disubstituted carbamoyl intermediates and the N-blocked benzyl intermediate. Thus, in a preferred embodiment of the invention, J in the above formula may be either

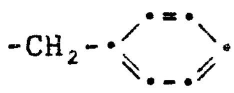 , 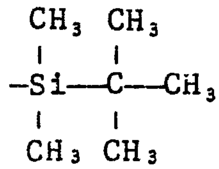 or 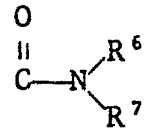 ,

wherein:

$R^6$ and $R^7$ are independently methyl, phenyl or

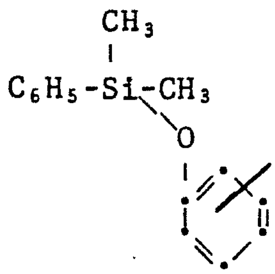 .

The 3-pyrazolidinones described above may be written in the keto form as:

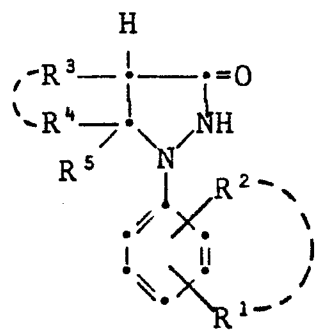

or in the enol form as:

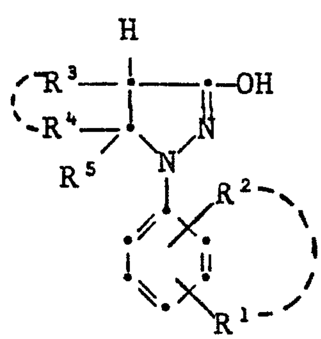

As used herein the keto form is meant to include the enol form and vice versa.

A common method for preparing the 3-pyrazolidinones described above involves reaction of a substituted hydrazine with a ß-halogenated or ß-hydroxy acid chloride or carboxylic acid. Variations of this method and other methods useful for preparing these compounds are detailed in U.S. Patents 2,289,367, 2,688,024, 2,704,762, 2,772,282, 2,843,598, 3,166,568, 3,330,839, and 4,074,051.

The second embodiment for preparing the blocked intermediate is formed by reacting a 3-chloro-2-pyrazoline having the structural formula:

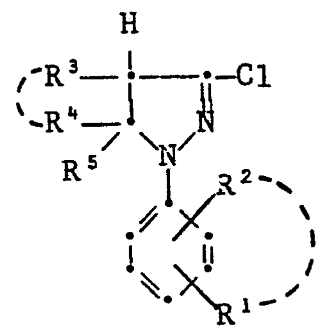

wherein:

$R^1$, $R^2$, $R^3$, $R^4$ and $R^5$ are defined as above, with a blocking group providing composition comprising potassium benzyloxide.

The 3-chloro-2-pyrazoline described above preferably is formed by reacting a 3-pyrazolidinone having the structural formula:

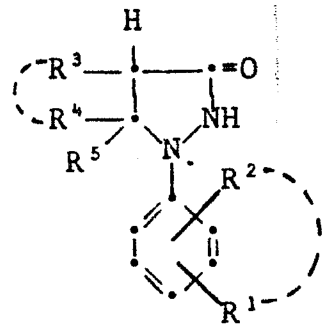

wherein:

$R^1$, $R^2$, $R^3$, $R^4$ and $R^5$ are defined as above, with an acid chloride such as phosgene in an inert solvent in the presence of a base. The reaction is advantageously completed in 1 to 24 hours at a temperature of 20°C to 70°C at about atmospheric pressure. This embodiment is preferred when the intermediate blocked by a benzyl group at the 3-position of the N-containing ring is prepared. This intermediate is desirable because the benzyl blocking group is more readily removable therefrom by hydrogenolysis after addition thereto of an electrophilic moiety as compared to the N-blocked-benzyl intermediates.

The intermediates described above must be stable at a temperature equal to or less than 0°C and in the presence of a strong base having a pKa greater than 20. Surprisingly, these blocked intermediates are stable under the harsh reaction conditions required to permit an electrophilic moiety to add to the C atom at the 4-position in the N-containing ring. These blocking groups can be removed after addition of the electrophilic moiety to form the desired 4-substituted-3-pyrazolidinone product in high yield.

When reacted with a 3-pyrazolidinone or a 2-pyrazoline, many blocking groups form intermediates

which are not stable at a temperature less than -20°C and in the presence of a strong base having a pKa greater than 20. For example, reacting trimethylsilylchloride with a 3-pyrazolidinone yields a trimethylsilyl iminoether blocked intermediate. However, this species does not survive the harsh reaction conditions required to permit an electrophilic moiety to selectively add to the C atom at the 4-position. Other blocking group providing compounds, such as acetylphenidone, are not operable despite formation of an intermediate stable at temperatures below 0°C in a strong base. This is because the initial blocking reaction yields a mixture of isomers and very harsh reaction conditions must be employed to remove the blocking group after alkylation has occurred, often resulting in decomposition of the newly formed species. Thus, useful blocking group providing compositions are those which will yield an intermediate stable not only at a temperature less than 0°C, but also in the presence of a strong base having a pKa greater than 20 and must also form an intermediate containing a blocking group that is readily removable.

As described above, the intermediate is reacted with an electrophilic moiety at a temperature less than 0°C in the presence of a strong base having a pKa greater than 20. When intermediates corresponding to the above structural formulas are formed in accordance with the preferred embodiment of the invention, an electrophilic moiety selectively adds to the C atom at the 4-position under the above described conditions. Electrophilic moieties useful herein are described in Advanced Organic Chemistry: Reactions, Mechanisms and Structure, by J. March, 2nd Ed., 1977, McGraw-Hill Book Co. The electrophilic moiety attaches to the C atom at the 4-position

through a single bond or through a double bond as follows:

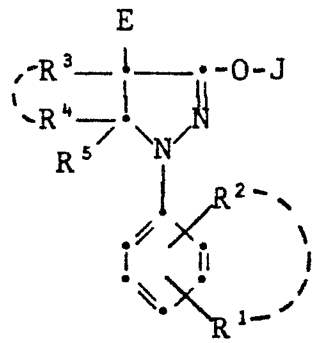

or

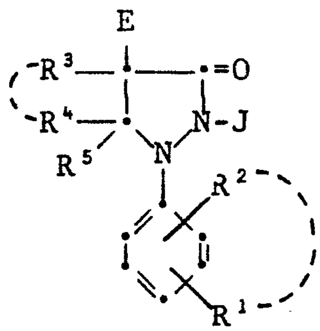

,

wherein E represents an electrophilic moiety, and $R^1$, $R^2$, $R^3$, $R^4$ and $R^5$ are defined as above.

The electrophilic moiety is provided by an electrophile reagent. Electrophile reagents are known to those skilled in the art. A partial listing of electrophile reagents useful in the practice of the invention comprises allyl and benzyl halides such as allyl chloride, allyl bromide, 1-bromo-4-methyl-but-2-ene, 1-bromo-but-2-ene (cis and/or trans), and benzyl bromide and substituted benzyl bromides;

alkyl halides such as methyl chloride, methyl bromide, methyl iodide, ethyl chloride, ethyl bromide, 1,2-dibromoethane, n-propylbromide, i-propyliodide, n-butyl iodide, 1-bromo-3-t-butyldimethylsilyloxy propane, n-hexyliodide and 2-bromohexane;

epoxides such as ethylene oxide, propylene oxide, isobutylene oxide, n-butylene oxide and 1,2-epoxyethylbenzene;

aldehydes such as acetaldehyde, formaldehyde, propionaldehyde, benzaldehyde, methoxyacetaldehyde, and dimethylaminoacetaldehyde;

ketones such as acetone, acetophenone, 2-butanone, cyclohexanone and dimethylaminoacetone;

acyl halides and derivatives such as acetyl chloride, benzoyl chloride, N,N-dimethylformamide, ethyl acetate and methyl chloroformamide;

chloromethyl ethers such as chloromethyl ether, chloromethyl isobutyl ether and chloromethoxy ether; and

silyl halides and derivatives such as trimethylsilyl chloride, t-butyl dimethylsilylchloride and chloromethyl trimethylsilane.

Certain electrophile providing reagents, recognized by those skilled in the art, such as acetyl chloride comprise divalent electrophile moieties. When such electrophile providing reagents are reacted with the intermediate, a first electrophilic moiety attaches to the 4-position of a first molecule of the intermediate. As a result, a second electrophile is revealed, which adds selectively to the 4-position of a second molecule of the intermediate. Thus, a single molecule of the electrophile providing reagent can react with 2 molecules of the intermediate. The term 'divalent electrophilic moiety' is used herein to describe such electrophilic moieties.

Examples of electrophilic moieties useful in the invention include:

$-CH_2-CH=CH_2$; $-CH_3$; $-\underline{i}C_3H_7$; $-\underline{n}C_4H_9$; $-CH_2O-CH(CH_3)_2$;

$-CH_2O(CH_2)_2OCH_3$; $-CH_2OCH_3$; $-CH_2CH_2OH$; $-(CH_2)_3OH$;

$-CH_2CH(CH_3)OH$; $-(CH_3)_2OH$; $-CH(OH)-CH(CH_3)_2$;

$-C(CH_3)_2OH$; $-CH(OH)-CH(CH_3)_2$; $-C(CH_3)(OH)(C_3H_5)$;

$-C(CH_2OH)_2OH$; $-CH(C_6H_5)OH$; $-C(CH_3)(C_6H_5)OH$;

$-C(CH_3)(OH)CH_2OCH_3$; $-C(CH_2OCH_3)_2OH$; $-CH_2N(CH_3)_2$;

$-SCH_3$; $-Si(CH_3)_3$; $-Si(CH_3)_2(\underline{t}-C_4H_9)$; $-CH_2Si(CH_3)_3$;

$-C(CH_3)(OH)CH_2OSi(CH_3)_2(\underline{t}-C_4H_9)$; $-CH_2OH$; $-CH(CH_3)OH$;

$-CH(C_2H_5)OH$; $-CH(OH)-CH(CH_3)_2$; $-CH_2OCH_2CH(CH_3)_2$;

$-(CH_2)_2CO_2CH_3$; $-CO_2CH_3$; 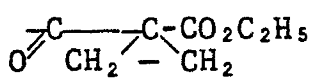; $-CHO$;

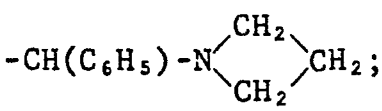; and $-(CH_2)OSi(CH_3)_2(\underline{t}-C_4H_9)$.

In practicing the process of the invention, a temperature equal to or below about 0°C is required to prevent side reactions from occuring which do not yield the desired product. At a temperature greater than about 0°C, the blocking group will tend to migrate from the O or N atom to which it must be attached as well as becoming detached later. The blocking group will form a C-C or C-Si bond depending on the particular blocking group used and the specific reaction conditions.

A strong base is required to permit the electrophilic moiety to add to the 4-position. The strong base removes the equivalent of $H^+$ from the 4-position, thereby permitting addition of the electrophilic moiety. Only strong bases having a pKa greater than about 20 remove the H atom attached to the 4-position. Strong bases having a pKa greater than about 20 useful in the practice of the invention include lithium or potassium diisopropylamide, n-butyllithium and the like.

The reaction mixture is stirred to completion, for a period of time generally less than 24 hours, and preferably from about 15 minutes to about 4 hours.

After completion of the reaction, the blocking group is removed. Blocking groups may be removed by hydrolysis, using reagents such as tetra-n-butylammonium fluoride in tetrahydrofuran, trifluoroacetic acid in aqueous tetrahydrofuran, or dilute hydrochloric acid. The removal of trialkylsilyl blocking groups by hydrolysis is described for example by R.F. Stewart and L.L. Miller in J. Amer. Chem. Soc., 102, p. 4999 (1980). Hydrolysis is preferably used to remove blocking groups derived from t-butyldimethylsilyl chloride and N,N-disubstituted carbamoyl chloride.

Alternatively, the blocking group can be removed by hydrogenolysis. Hydrogenolysis comprises removal of the blocking group using hydrogen gas. Preferably, hydrogenolysis is carried out in the presence of a catalyst such as commercially available palladium on carbon catalyst. A detailed description of hydrogenolysis, useful herein to remove blocking groups, is described in Advanced Organic Chemistry: Reactions, Mechanisms and Structure by J. March, 2nd Ed., 1977, McGraw-Hill Book Co. Hydrogenolysis is

preferably used to remove blocking groups derived from potassium benzyloxide and benzyl bromide.

With the removal of the blocking groups, the 4-substituted-3-pyrazolidinones prepared by the process of the invention have the following formula:

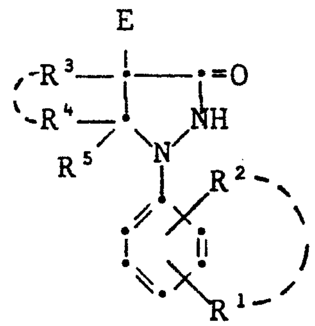

$R^1$, $R^2$, $R^3$, $R^4$, $R^5$ and E being defined as above.

Compounds prepared by the process of the invention include the following:

Table I

The following compounds are among those that have been prepared from 1-phenyl-3-pyrazolidinone or its p-tolyl, 4'-methoxyphenyl, 3',4'-dimethoxyphenyl or 3',4'-methylenedioxyphenyl analog. The electrophile reagent used to add the group E in the synthesis is also given.

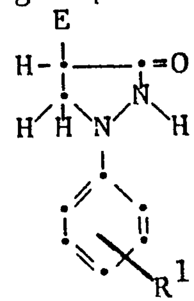

| Compound | Electrophile Reagent | E | $R^1$ |
|---|---|---|---|
| 1 | $CH_2=CHCH_2Br$ | $-CH_2-CH=CH_2$ | -H |
| 2 | $CH_2=CHCH_2Br$ | $-CH_2-CH=CH_2$ | 4'-$CH_3$ |
| 3 | $CH_3I$ | $-CH_3$ | 3',4'-$OCH_2O$- |
| 4 | $CH_3I$ | $-CH_3$ | 3',4'-$(-OCH_3)_2$ |
| 5 | $(CH_3)_2CHI$ | i$C_3H_7$ | -H |

Table I (cont'd)

| Compound | Electrophile Reagent | E | $R^1$ |
|---|---|---|---|
| 6 | $(CH_3)_2CHI$ | <u>i</u>$C_3H_7$ | 4'-$OCH_3$ |
| 7 | $(CH_3)_2CHI$ | <u>i</u>$C_3H_7$ | 4'-F |
| 8 | <u>n</u>$C_4H_9I$ | <u>n</u>$C_4H_9$ | -H |
| 9 | $(CH_3)_2CHOCH_2Cl$ | $-CH_2O-CH(CH_3)_2$ | 4'-$CH_3$ |
| 10 | $CH_3O(CH_2)_2OCH_2Cl$ | $-CH_2O(CH_2)_2OCH_3$ | 4'-$OCH_3$ |
| 11 | $CH_3OCH_2Cl$ | $-CH_2OCH_3$ | 4'-$OCH_3$ |
| 12 | 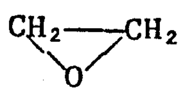 | $-CH_2CH_2OH$ | 4'-$OCH_3$ |
| 13 | <u>t</u>-$C_4H_9(CH_3)_2SiO(CH_2)_3Br$ | $-(CH_2)_3OH$ | 4'-$CH_3$ |
| 14 | 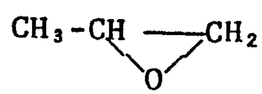 | $-CH_2CH(CH_3)OH$ | 4'-$CH_3$ |
| 15 | $CH_3COCH_3$ | $-C(CH_3)_2OH$ | 4'-$CH_3$ |

Table I (cont'd)

| Compound | Electrophile Reagent | E | $R^1$ |
|---|---|---|---|
| 16 | $CH_3COCH_3$ | $-C(CH_3)_2OH$ | 4'-$OCH_3$ |
| 17 | $CH_3COCH_3$ | $-C(CH_3)_2OH$ | 3',4'-$OCH_2O$- |
| 18 | $(CH_3)_2CHCHO$ | $-CH(OH)-CH(CH_3)_2$ | 4'-$CH_3$ |
| 19 | $C_3H_5COCH_3$ | $-C(CH_3)(OH)(C_3H_5)$ | 4'-$CH_3$ |
| 20 | $(\underline{t}-C_4H_9(CH_3)_2SiOCH_2)_2\rangle C=O$ | $C(CH_2OH)_2OH$ | 4'-$OCH_3$ |
| 21 | $C_6H_5CHO$ | $-CH(C_6H_5)OH$ | 4'-$CH_3$ |
| 22 | $C_6H_5COCH_3$ | $-C(CH_3)(C_6H_5)OH$ | 4'-$CH_3$ |
| 23 | $CH_3OCH_2COCH_3$ | $-C(CH_3)(OH)CH_2OCH_3$ | 4'-$CH_3$ |
| 24 | $CH_3OCH_2COCH_3$ | $-C(CH_3)(OH)CH_2OCH_3$ | 4'-$OCH_3$ |

Table I (cont'd)

| Compound | Electrophile Reagent | E | $R^1$ |
|---|---|---|---|
| 25 | $(CH_3OCH_2)_2C=O$ | $-C(CH_2OCH_3)_2OH$ | $4'-OCH_3$ |
| 26 | $CH_2=\overset{+}{N}(CH_3)_2I^-$ | $-CH_2N(CH_3)_2$ | -H |
| 27 | $CH_3-S-S-CH_3$ | $-SCH_3$ | $4'-CH_3$ |
| 28 | $CH_3COCH_2N(CH_3)_2$ | $-C(CH_3)(OH)CH_2N(CH_3)_2$ | $4'-CH_3$ |
| 29 | $(CH_3)_3SiCl$ | $-Si(CH_3)_3$ | $4'-OCH_3$ |
| 30 | $(CH_3)_2(\underline{t}-C_4H_9)SiCl$ | $-Si(CH_3)_2(\underline{t}-C_4H_9)$ | $4'-CH_3$ |
| 31 | $(CH_3)_3SiCH_2Cl$ | $-CH_2Si(CH_3)_3$ | $4'-CH_3$ |
| 32 | $\underline{t}-C_4H_9(CH_3)_2SiOCH_2-COCH_3$ | $-C(CH_3)(OH)CH_2OSi(CH_3)_2(\underline{t}-C_4H_9)$ | $4'-CH_3$ |
| 33 | $Br-CH_2-CH_2-Br$ | 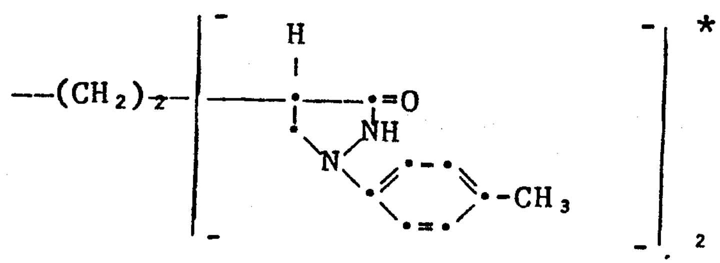 | |

*This represents the entire structure including E and $R^1$.

## Table II

The following 4-(α-hydroxyalkyl) compounds are among those that have been prepared from 4-alkyl-1-phenyl-3-pyrazolidinones or phenyl-substituted analogs.

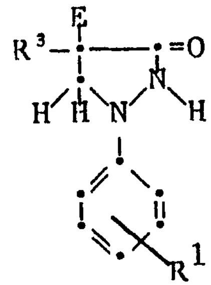

| Compound | Electrophile Reagent | E | $R^3$ | $R^1$ |
|---|---|---|---|---|
| 34 | $CH_2O$ | $-CH_2OH$ | $-CH_3$ | 4'-F |
| 35 | $CH_2O$ | $-CH_2OH$ | $-CH_3$ | 3',4'-$OCH_2O$- |
| 36 | $CH_2O$ | $-CH_2OH$ | $\underline{i}C_3H_7$ | 4'-$OCH_3$ |
| 37 | $CH_2O$ | $-CH_2OH$ | $\underline{n}C_4H_9$ | -H |
| 38 | $CH_2O$ | $-CH_2OH$ | 4,5-$(CH_2)_4$- | 4'-$CH_3$ |
| 39 | $CH_2O$ | $-CH_2OH$ | $-CH_2-CH=CH_2$ | 4'-$CH_3$ |

Table II (cont'd)

| Compound | Electrophile Reagent | E | $R^3$ | $R^1$ |
|---|---|---|---|---|
| 40 | $CH_3CHO$ | $-CH(CH_3)OH$ | $-CH_3$ | -H |
| 41 | $C_2H_5CHO$ | $-CH(C_2H_5)OH$ | $-CH_3$ | -H |
| 42 | $(CH_3)_2CHCHO$ | $-CHOH-CH(CH_3)_2$ | $-CH_3$ | $4'-CH_3$ |
| 43 | $C_6H_5CHO$ | $-CH(C_6H_5)OH$ | $-CH_3$ | $4'-CH_3$ |
| 44 | $CH_3COCH_3$ | $-C(CH_3)_2OH$ | $-CH_3$ | $4'-OCH_3$ |
| 45 | $C_6H_5COCH_3$ | $-C(CH_3)(C_6H_5)OH$ | $-CH_3$ | $4'-CH_3$ |
| 46 | $CH_3COCl$ | 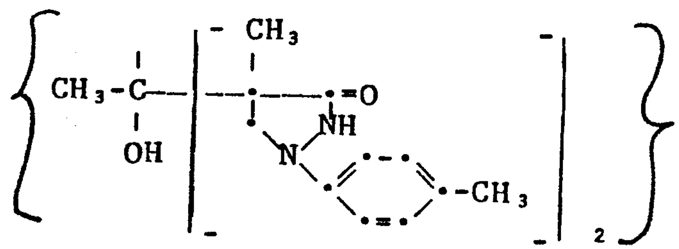 * | | |

*This represents the entire structure including E and $R^1$.

Table II (cont'd)

| Compound | Electrophile Reagent | E | $R^3$ | $R^1$ |
|---|---|---|---|---|
| 47 | $CH_2=CHCH_2Br$ | $-CH_2-CH=CH_2$ | $-CH_3$ | -H |
| 48 | $CH_3I$ | $-CH_3$ | $4,5-CH_2CH_2O$ | $4'-CH_3$ |
| 49 | $\underline{n}-C_4H_9I$ | $\underline{n}-C_4H_9$ | $\underline{n}-C_4H_9$ | -H |
| 50 | $\underline{n}-C_4H_9I$ | $\underline{n}-C_4H_9$ | $-CH_3$ | $4'-CH_3$ |
| 51 | 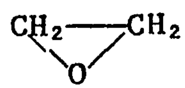 | $-(CH_2)_2OH$ | $-CH_3$ | $4'-CH_3$ |
| 52 | 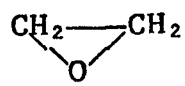 | $-(CH_2)_2OH$ | $-CH_2CH=CH_2$ | $4'-CH_3$ |
| 53 | 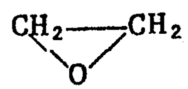 | $-(CH_2)_2OH$ | $-CH_3$ | -H |
| 54 | 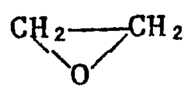 | $-(CH_2)_2OH$ | $4,5-(CH_2)_3$ | -H |

Table II (cont'd)

| Compound | Electrophile Reagent | E | $R^3$ | $R^1$ |
|---|---|---|---|---|
| 55 | 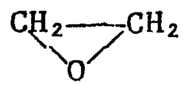 | $-(CH_2)_2OH$ | $\underline{i}C_3H_7$ | -H |
| 56 | 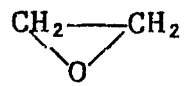 | $-(CH_2)_2OH$ | $-CH_3$ | 3',4'-$CH_3$ |
| 57 | $\underline{t}$-$C_4H_9(CH_3)_2SiO(CH_2)_3Br$ | $-(CH_2)_3OH$ | $-CH_3$ | 4'-$CH_3$ |
| 58 | $\underline{t}$-$C_4H_9(CH_3)_2SiO(CH_2)_3Br$ | $-(CH_2)_2OH$ | $-(CH_2)_3OH$ | 4'-$CH_3$ |
| 59 | 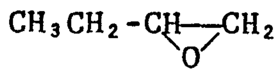 | $-CH_2CH(C_2H_5)OH$ | $-CH_3$ | 4'-$CH_3$ |
| 60 | 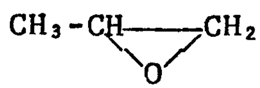 | $-CH_2CH(CH_3)OH$ | $-CH_3$ | 4'-$CH_3$ |
| 61 | $(CH_3)_2CHOCH_2Cl$ | $-CH_2OCH_2CH(CH_3)_2$ | $-CH_3$ | 4'-$CH_3$ |
| 62 | $CH_2=CHCO_2CH_3$ | $-(CH_2)_2CO_2CH_3$ | $-CH_3$ | -H |
| 63 | $CH_3OCOCl$ | $-CO_2CH_3$ | $-CH_3$ | 4'-$CH_3$ |

## Table II (cont'd)

| Compound | Electrophile Reagent | E | $R^3$ | $R^1$ |
|---|---|---|---|---|
| 64 | 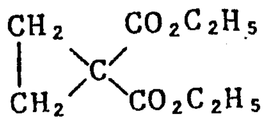 | 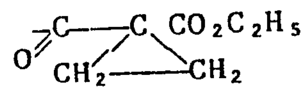 | $-CH_3$ | $4'-CH_3$ |
| 65 | $(CH_3)_2NCHO$ | $-CHO$ | $-CH_3$ | $4'-CH_3$ |
| 66 | $CH_3-S-S-CH_3$ | $-SCH_3$ | $-SCH_3$ | $4'-CH_3$ |
| 67 | $CH_2=N^+(CH_3)_2I^-$ | $-CH_2N(CH_3)_2$ | $-CH_3$ | $4'-OCH_3$ |
| 68 | $CH_2=N^+(CH_3)_2I^-$ | $-CH_2N(CH_3)_2$ | $\underline{i}C_3H_7$ | $4'-CH_3$ |
| 69 | $Br(CH_2)_3-N=CH-C_6H_5$ | 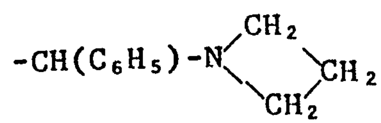 | $-CH_3$ | $4'-CH_3$ |

<u>Table II (cont'd)</u>

| Compound | Electrophile Reagent | E | $R^3$ | $R^1$ |
|---|---|---|---|---|
| 70 | $(CH_3)_2(\underline{n}-C_4H_9)SiCl$ | $-Si(CH_3)_2(\underline{t}-C_4H_9)$ | $4,5\ -(CH_2)_3-$ | $3',4'-CH_3$ |
| 71 | $(CH_3)_3SiCl$ | $-Si(CH_3)_3$ | $-CH_3$ | $3',4'-CH_3$ |
| 72 | 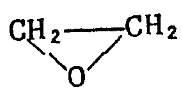 | $-(CH_2)_2OH$ | $-Si(CH_3)_3$ | $4'-CH_3$ |
| 73 | $CH_2O$ | $-(CH_2)OSi(CH_3)_2(\underline{t}-C_4H_9)$ | $4,5-(CH_2)_4-$ | $-H$ |

## Table III

The following 4,4-alkene compounds are among those that have been prepared from 1-aryl-4-trimethylsilyl-3-pyrazolidinones.

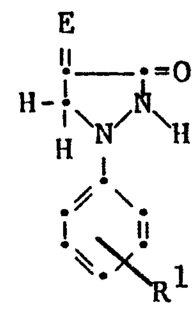

| Compound | Electrophile Reagent | E | $R^1$ |
|---|---|---|---|
| 74 | $CH_3COCH_3$ | ‖<br>$CH_3-C-CH_3$ | -H |
| 75 | $CH_3COCH_2N(CH_3)_2$ | ‖<br>$CH_3-C-CH_2-N(CH_3)_2$ | 4'-$CH_3$ |
| 76 | $CH_3CO-CH—CH_2$<br>$CH_2$ | ‖ H<br>$CH_3-C-C—CH_2$<br>$CH_2$ | 4'-$CH_3$ |
| 77 | t-$C_4H_9(CH_3)_2-SiOCH_2COCH_3$ | ‖<br>$CH_3-C-CH_2OH$ | 4'-$CH_3$ |
| 78 | t-$C_4H_9(CH_3)_2SiOCH_2)_2$ >C=O | ‖<br>$HOCH_2-C-CH_2OH$ | 4'-$CH_3$ |

The following preparations and examples are included to illustrate the practice of this invention.

Example 1 - Preparation of 4-(2-hydroxyethyl)-4-methyl-1-phenyl-3-pyrazolidinone (Compound 53)

To a stirred solution under nitrogen of 4-methyl-1-phenyl-3-pyrazolidinone (17.6 g, 0.10 mole), t-butyldimethylsilyl chloride (16.5 g, 0.11 mole), in 200 ml toluene and 25 ml anhydrous tetrahydrofuran was added sequentially triethylamine (11.0 g, 0.11 mole), N,N-dimethylaminopyridine (0.1 g) and 1,8-diazabicyclo [5.4.0] undec-7-ene (0.1 g). The mixture was refluxed for 16 hours, then cooled to 30°C, and filtered. After the solids were washed with ether, the solvent of the combined filtrate was removed under reduced pressure. The resulting solid was heated with 150 ml anhydrous ether and filtered to remove salts. The filtrate was dried over anhydrous magnesium sulfate and freed of solvent to yield 29.0 g (100%) of 4-methyl-1-phenyl-3-(t-butyldimethylsiloxy)-2-pyrazoline.

A stirred solution at 0°C under nitrogen of diisopropylamine (2.35 g, 0.023 mole) in 20 ml anhydrous tetrahydrofuran was treated with n-butyl lithium (14.5 ml of a 1.6 M solution in hexane). The mixture was allowed to stir at 0°C for 15 minutes and was then cooled to -78°C using a dry ice-acetone bath. To this mixture a solution of 4-methyl-1-phenyl-3-(t-butyldimethylsiloxy)-2-pyrazoline (5.8 g, 0.020 mole) in 50 ml anhydrous tetrahydrofuran was added dropwise over a five minute period. After the mixture was stirred for 25 minutes at -78°C, an excess of ethylene oxide (3.0 g, 0.070 mole) was added in one portion, and the mixture was allowed to warm to room temperature. The mixture was then treated with 20 ml saturated aqueous ammonium chloride, the organic layer was separated and washed with 100 ml saturated aqueous

sodium chloride. The solution was dried over anhydrous magnesium sulfate and freed of solvent.

The residue was suspended in 50 ml anhydrous tetrahydrofuran and treated at room temperature with stirring under nitrogen with tetra-n-butylammonium fluoride (20 ml of 1 M solution in tetrahydrofuran). After two minutes reaction, the mixture was freed of most solvent under reduced pressure. The residue slurry was passed through a silica column and eluted with a (95:5:5) mixture of methylene chloride, ether, and ethanol. The appropriate fractions were united, and freed of solvent. The resulting oil was crystallized from hot ethanol to yield 3.0 g (70% yield) of desired 4-(2-hydroxyethyl)-4-methyl-1-phenyl-3-pyrazolidone. Analysis was confirmed by infrared and NMR spectra.

Example 2 - Preparation of 4-isopropylidene-1-phenyl-3-pyrazolidinone (Compound 74)

A stirred solution under nitrogen of 4-trimethylsilyl-1-phenyl-3-pyrazolidinone (4.7 g, 0.20 mole), t-butyldimethylsilyl chloride (3.2 g, 0.21 mole), and triethylamine (2.0 g, 0.20 mole), in 50 ml anhydrous toluene was refluxed for two hours. The mixture was cooled to room temperature and filtered. The solids were washed with 100 ml anhydrous ether, the combined filtrate was dried over anhydrous sodium sulfate, and the solvent was removed under reduced pressure. The residue was triturated with 100 ml anhydrous ether and filtered to remove salts. The filtrate was dried over anhydrous magnesium sulfate and freed of solvent under vacuum to yield 6.8 g (98%) of 4-trimethylsilyl-1-phenyl-3-(t-butyldimethylsiloxy)-2-pyrazoline. Analysis was confirmed by NMR spectra.

A stirred solution at 0°C under nitrogen of diisopropylamine (1.21 g, 0.012 mole) in 40 ml anhydrous tetrahydrofuran was treated with n-butyl

lithium (7.5 ml of a 1.6 M solution in hexane). The mixture was allowed to stir at 0°C for 15 minutes and was then cooled to -78°C. To this mixture a solution of 4-trimethylsilyl-1-phenyl-3-(t-butyldimethylsiloxy)-2-pyrazoline (3.5 g, 0.010 moles) in 60 ml anhydrous tetrahydrofuran was added dropwise over a five minute period. The resulting amber solution was stirred for 25 minutes at -78°C, then treated with excess anhydrous acetone in one portion, and stirred an additional 20 minutes at -78°C. After warming to room temperature, 20 ml saturated aqueous ammonium chloride was added, and the mixture was diluted with 100 ml each of ether and ethyl acetate. After the aqueous layer was separated and extracted with 100 ml ethyl acetate, the combined organic phases were washed with 20 ml ice-cold 10% aqueous hydrochloric acid. The organic layer was separated, dried over anhydrous magnesium sulfate and freed of solvent under reduced pressure. The resulting solid was triturated with anhydrous ether, filtered, and air-dried to yield 1.38 g (68.2%) of 4-isopropylidene-1-phenyl-3-pyrazolidinone. Analysis was confirmed by $^{1}$HNMR $^{13}$CNMR and infrared spectra.

Example 3 - Comparative Example - Preparation of 4-(3-hydroxypropyl)-1-phenyl-3-pyrazolidinone - by Prior Art Process

This example compares a prior art process to the process of the present invention.

## The Prior Art Process

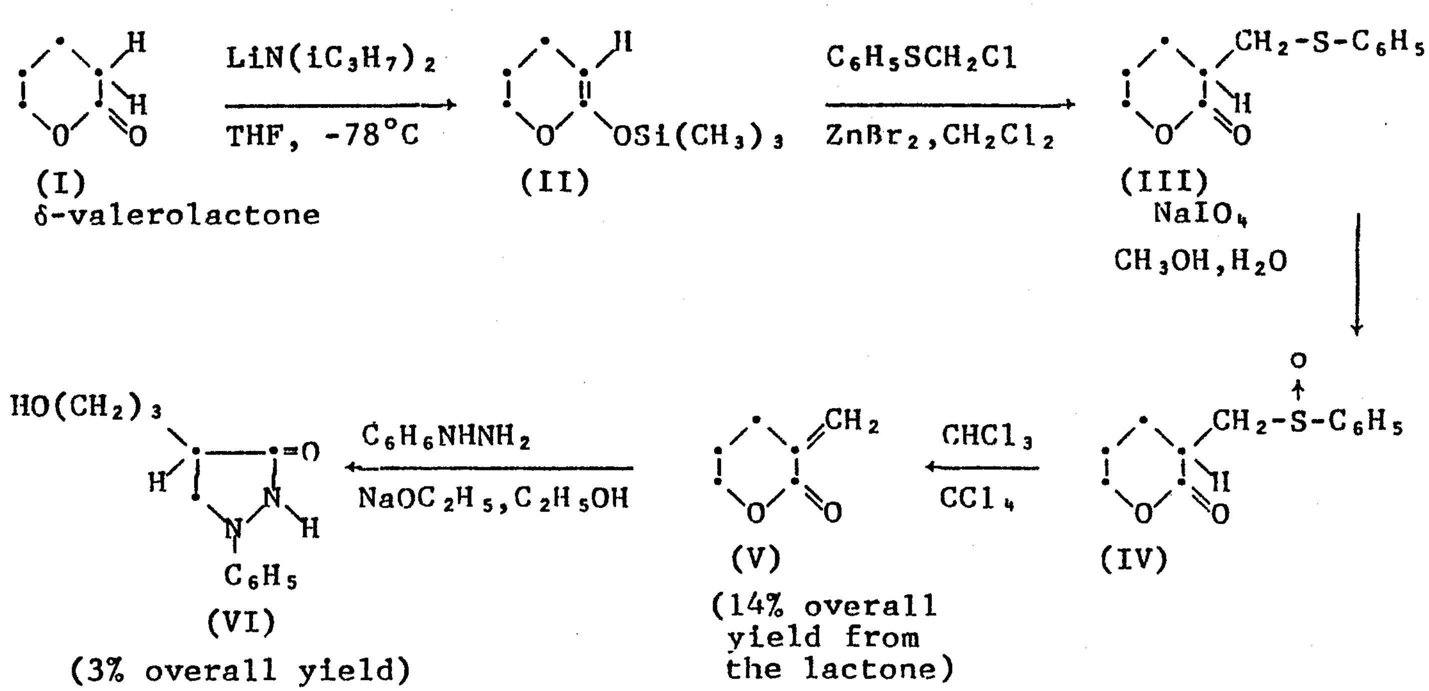


(I) δ-valerolactone

(II)

(III)

(IV)

(V) (14% overall yield from the lactone)

(VI) (3% overall yield)

Preparation of (II)

A stirred solution at 0°C under nitrogen of diisopropylamine (20. g, 0.20 mmole) in 150 ml anhydrous tetrahydrofuran was treated with n-butyllithium (14 g, 0.22 mole) in 90 ml n-hexane and stirred for 15 minutes at 0°C. The solution was then cooled to -78°C, treated dropwise over a period of 10 minutes with a solution of valerolactone (I) (20 g, 0.2 mole) in 50 ml anhydrous tetrahydrofuran, and allowed to stir at -78°C for 30 minutes. Trimethylsilyl chloride (50 g, 0.46 mole) was then added dropwise over a period of 10 minutes, and the solution allowed to stir at -78°C for 35 minutes. After warming to room temperature the mixture was stirred for an additional hour. The mixture was filtered; the filtrate was concentrated under reduced pressure, triturated with anhydrous ether, filtered, and all solvent was removed under reduced pressure. The residue was distilled at reduced pressure to yield 29 g solid (84% yield). Analysis of II was confirmed by NMR spectra.

Preparation of (III)

To a stirred solution at room temperature under nitrogen of chloromethyl phenyl sulfide (9.5 g, 0.060 mole) in 50 ml dichloromethane was added in one portion Compound (II) (8.6 g, 0.050 mole) in 50 ml dichloromethane. The solution was treated with zinc bromide (2.3 g, 0.010 mole) and allowed to stir for 18 hours at room temperature. After removal of solvent under reduced pressure, the residue was chromatographed on silica using 3:2 hexane:ethyl acetate as the eluting solvent. The yield of III was 6.3 g (56%). Analysis of III was confirmed by NMR spectra.

Preparation of (IV)

A stirred ice-cold solution under nitrogen of sodium periodate (7.0 g, 0.033 mole) in 150 ml water was treated with Compound (III) (6.3 g, 0.028 mole) in

200 ml methanol and 40 ml cyclohexane, and allowed to stir an additional 18 hours. The mixture was then extracted with four 125 ml portions of dichloromethane. The combined extracts were washed with three 500 ml portions of water, dried over anhydrous magnesium sulfate and the solvent was removed under reduced pressure. The residue was chromatographed on silica using 1:1 dichloromethane:ether as the eluting solvent. The yield of the sulfoxide (IV) was 1.9 g (38%). Analysis of IV was confirmed by NMR spectra.

Preparation of (V)

A stirred solution of IV (1.1 g, 0.046 mole) in 5 ml chloroform and 5 ml carbon tetrachloride was refluxed for 4.5 hours. The solution was freed of solvent under reduced pressure and the residue was 'bulb to bulb' distilled to yield 0.45 g (87% yield) of V, as a clear colorless oil, bp 100°C, 20 mm. Analysis of V was confirmed by NMR spectra.

Preparation of (VI)

Under nitrogen at room temperature, phenylhydrazine (0.43 g, 4.0 mmole) was added to a solution of sodium ethoxide (1.0 g, 16 mmole) in 10 ml ethanol. The stirred mixture was treated with Compound (V) (0.45 g, 4.0 mmole) in 2 ml ethanol and refluxed for 14 hours. The solution was cooled to room temperature, treated with 2 ml glacial acetic acid, extracted with two 75 ml portions of dilute aqueous sodium bicarbonate, dried over magnesium sulfate. The solvent was removed under reduced pressure and the residue was chromatographed on silica using a mixture of dichloromethane, ethyl ether, and ethanol as the eluting solvent. The yield of (VI), 4-(3-hydroxypropyl)-1-phenyl-3-pyrazolidinone was 0.25 g (28%). Analysis was confirmed by $^{1}$HNMR and $^{13}$CNMR spectra. Overall yield based on (I) was 3.0%.

Preparation of 4-(3-hydroxypropyl)-1-p-tolyl-3-pyrazolidinone By the Process of the Present Invention

To a stirred solution under nitrogen of 1-p-tolyl-3-pyrazolidinone (52.8 g, 0.30 mole) in 500 ml toluene and 50 ml anhydrous tetrahydrofuran was added sequentially t-butyldimethylsilyl chloride (49.5 g, 0.30 mole), triethylamine (33.0 g, 0.30 mole), 4-(N,N-dimethylamino)pyridine (1.0 g) and 1,8-diazabicyclo[5.4.0]-undec-7-ene (0.1 g). The mixture was refluxed for 3 hours, then cooled to 30°C, and filtered. After the solids were washed with ether, the solvents of the combined filtrate were removed under reduced pressure. The resulting solid was dissolved in 500 ml anhydrous ether and filtered to remove salts. Removal of the solvent of the filtrate yielded 85.6 g (98% yield) of 1-p-tolyl-3-(t-butyldimethylsiloxy)-2-pyrazoline.

To a stirred room-temperature solution under nitrogen of t-butyldimethylsilyl chloride (49.7 g, 0.30 mole) and imidazole (51.0 g, 0.75 mole) in 100 ml N,N-dimethylformamide was added in one portion 3-bromopropanol (41.7 g, 0.30 mole). The slightly exothermic reaction mixture was stirred for 3 hours, diluted with ice-water, and extracted three times with 200 ml portions of ether. The combined ether extracts were washed five times with 100 ml portions of water and dried over anhydrous magnesium sulfate. After removal of solvent, the residue was distilled under reduced pressure to yield 43.0 g of a clear oil, 3-bromo-1-(t-butyldimethylsiloxy)propane.

A solution of 1-p-tolyl-3-(t-butyldimethylsiloxy)-2-pyrazoline (11.6 g, 0.040 mole), prepared as in two paragraphs above, in 80 ml anhydrous tetrahydrofuran was added dropwise over a 10 minute period to a stirred solution at -78°C of lithium diisopropylamide in 50 ml anhydrous tetrahydrofuran

under nitrogen. The lithium diisopropylamide was generated at 0°C by mixing diisopropylamine (5.1 g) and 20.8 ml of a 2.4 M solution of n-butyllithium in n-hexane. The resulting orange-red solution was allowed to stir for 30 minutes at -78°C, and was then treated with a single portion of 3-bromo-1-(t-butyldimethylsiloxy)propane (10 g, 0.040 mole) dissolved in 40 ml anhydrous tetrahydrofuran. After being allowed to warm to room temperature overnight, the mixture was diluted with 150 ml ether and 100 ml 10% aqueous hydrochloric acid and shaken vigorously. The separated aqueous layer was extracted with a (1:1) mixture of ether and ethyl acetate. The combined extracts were dried over magnesium sulfate. After removal of solvent the residue was chromatographed on a silica column and eluted with (70:30) hexane:ethyl acetate. A first fraction of 5.0 g (representing "4-monoalkylated" product), and third fraction of 2.8 g of product 4-(3-hydroxypropyl)-1-p-tolyl-3-pyrazolidinone were obtained. A second fraction of 1.0 g of "4,4-dialkylated" material was discarded. A total yield of 66% could be considered after hydrolysis of the first fraction.

The first fraction (1.5 g) was dissolved in 100 ml tetrahydrofuran, stirred under nitrogen at room temperature, and heated sequentially with 10 ml water and 0.5 ml trifluoroacetic acid. After two hours stirring the mixture was diluted with 200 ml ether, washed sequentially with water, ice cold saturated aqueous sodium bicarbonate, and saturated aqueous sodium chloride, and then dried over anhydrous magnesium sulfate. After removal of solvent, the oily residue was chromatographed on a silica column and eluted with (80:20:2) methylene chloride, ether, ethanol. After recrystallization from 1:1 toluene and ether, 0.9 g of product 4-(3-hydroxypropyl)-1-p-tolyl-3-pyrazolidinone was obtained. Analysis was confirmed by NMR spectra.

This example illustrates that a significantly higher yield is obtained (66%) using the process of the invention as compared to the prior art process (3%). The substitution of a methyl group in the 4'-position is a trivial difference not affecting the basic preparations.

Example 4 - Utility of 4-Substituted-3-pyrazolidinones as Electron Transfer Agents (ETA's) in Integral Imaging Receiving Element

To evaluate Compounds A, B and C with respect to their effectiveness as an ETA in an integral imaging receiving element, the following photographic element was prepared by coating the following layers in the order recited on a transparent poly(ethylene terephthalate) film support. Quantities are parenthetically given in g/m², unless otherwise stated.

(1) image-receiving layer of a poly(divinylbenzene-co-styrene-co-N-benzyl-N,N-dimethyl-N-vinylbenzyl)ammonium sulfate latex mordant (2.3) and gelatin (2.3);

(2) reflecting layer of titanium dioxide (16) and gelatin (2.6);

(3) opaque layer of carbon black (1.9) and gelatin (1.2);

(4) cyan dye-providing layer of gelatin (1.2), cyan RDR (0.3), dispersed in 1,4-cyclohexylenedimethylene bis(2-ethylhexanoate) and gelatin (0.7);

(5) red-sensitive, direct-positive silver bromide emulsion (silver - 0.9, gelatin - 0.9), 1-[4-(2-formylhydrazino)phenyl]-3-methylthiourea (0.01), 2-(2-octadecyl-5-sulfohydroquinone) potassium salt (0.2), and aceto-2-p-[5-amino-2-(2,4-di-t-pentylphenoxy)benzamido]phenyl hydrazide (0.1);

(6) interlayer of gelatin (1.6) and 2,5-di-sec-dodecylhydroquinone (1.3);

(7) magenta dye-providing layer of magenta RDR (0.3) dispersed in 1,4-cyclohexylenedimethylene-bis(2-ethylhexanoate) and gelatin (0.7);

(8) green-sensitive, direct-positive silver bromide emulsion (silver - 0.9, gelatin - 0.9), 1-[4-(2-formylhydrazino)phenyl]-3-methylthiourea (0.01), aceto-2-[5-amino-2-(2,4-di-t-pentyl-phenoxy)benzamido]phenyl hydrazide (0.4), and 2-(2-octadecyl-5-sulfohydroquinone) potassium salt (0.2);

(9) interlayer of gelatin (1.6) and 2,5-di-sec-dodecylhydroquinone (1.3);

(10) yellow dye-providing layer of yellow RDR (0.4) dispersed in 1,4-cyclohexylenedimethylene-bis(2-ethyl-hexanoate) and gelatin (0.7);

(11) blue-sensitive, direct-positive silver bromide emulsion (silver - 0.9, gelatin - 0.9), 1-[4-(2-formylhydrazino)phenyl]-3-methylthiourea (0.005), aceto-2-p-[5-amino-2-(2,4-di-t-pentylphenoxy)benzamido]-phenyl hydrazide (0.2), and 2-(2-octadecyl-5-sulfohydroquinone) potassium salt (0.2); and

(12) overcoat layer of gelatin (0.9).

<u>Compound A</u>

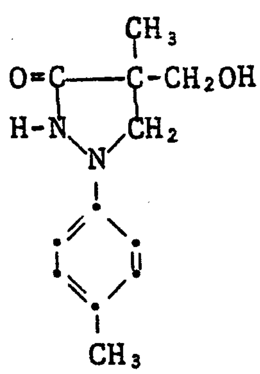

Compound B

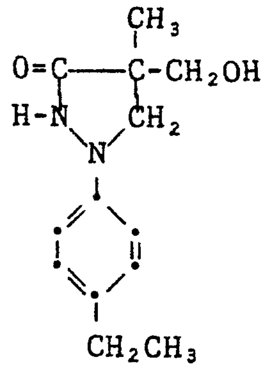

Compound C

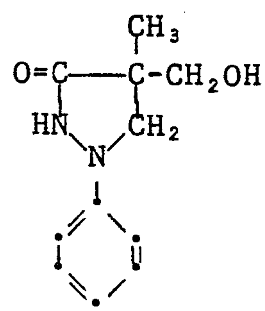

Cyan RDR

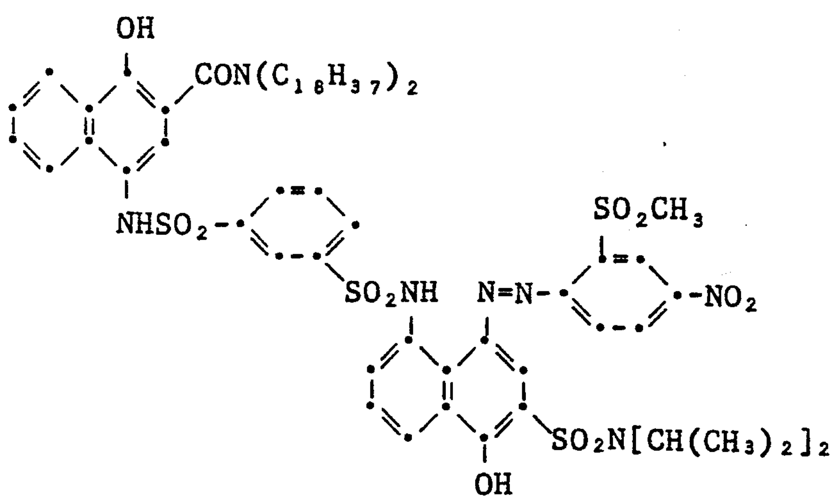

Magenta RDR

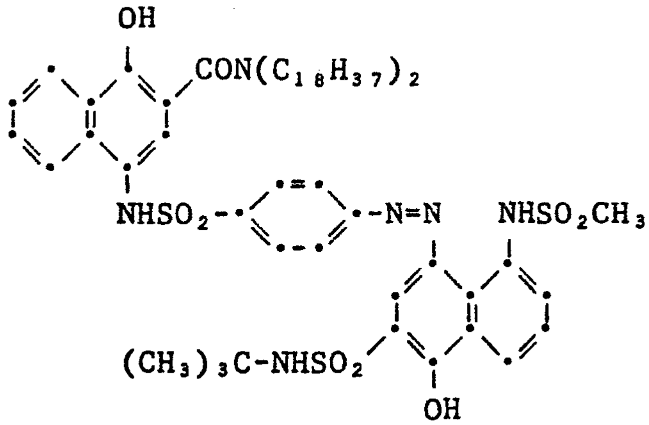

Yellow RDR

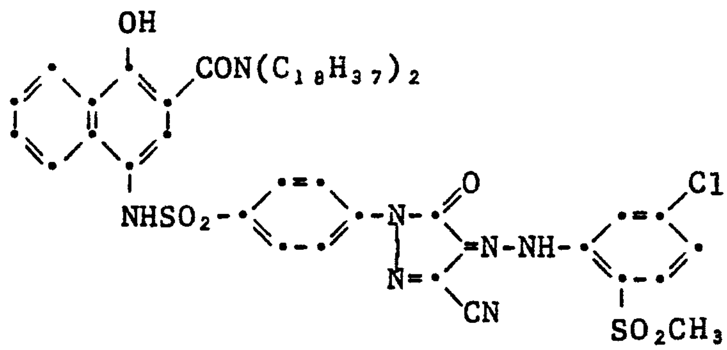

Samples of the above-prepared photosensitive elements were exposed through a multicolor graduated-density test object. The exposed samples were then processed under dark conditions at 72°F. (22°C.) by rupturing a pod containing the viscous processing compositions described below between the photosensitive element and a transparent cover sheet, as described below.

The processing compositions were as follows:

| Processing Composition A | | |
|---|---|---|
| Potassium hydroxide (45 percent aqueous solution) | 104 | g |
| Sodium sulfite | 1.0 | g |
| 5-Methylbenzotriazole | 4 | g |
| ETA (Compound C) | 12.0 | g |
| 1,4-Cyclohexanedimethanol | 1 | g |
| Sodium salt of naphthalene-formaldehyde condensate | 6.4 | g |
| Potassium fluoride • $2H_2O$ | 10.0 | g |
| Carboxymethylcellulose | 44.0 | g |
| Water to 1 liter | | |

Processing Composition B

Same as A, except that the ETA was Compound A at an equimolar concentration of 12.8 grams per liter.

Processing Composition C

Same as A, except that the ETA was Compound B at an equimolar concentration of 13.6 grams per liter.

The cover sheet consisted of a transparent poly(ethylene terephthalate) film support having coated thereon:

(1) a polyacrylic acid layer (17.5 meq/0.093 $m^2$).

(2) a timing layer comprising a polymeric mixture as disclosed in U.S. Patent No. 4,229,516 issued October 21, 1980 to Abel entitled "Photographic Material with Temporary Barrier Layer Comprising a Mixture of Vinylidene Chloride Terpolymer and Polymeric Carboxy-Ester-Lactone and Photographic Transfer Process Therefor".

Initial access times, which are defined as the time that the transferred dye image is first visible to the eye, were determined for each of the elements as follows:

| Processing Composition | ETA | Initial Access Time (seconds) |
|---|---|---|
| A | Compound C | 52 |
| B | Compound A | 31 |
| C | Compound B | 26 |

The above examples indicate that 4-substituted-3-pyrazolidinones are useful as ETAs in integral imaging receiving elements.

CLAIMS:

1. A process for the preparation of 4-substituted-3-pyrazolidinones characterized by:

(1) reacting

A) a compound having the structural formula:

(i) 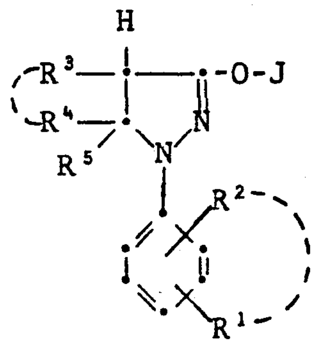 or

(ii) 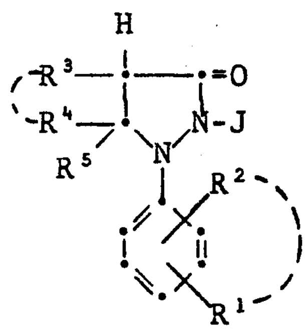

wherein:

$R^1$, $R^2$, $R^3$, $R^4$ and $R^5$ are independently hydrogen; fluorine; allyl; trimethylsilyl; benzyl; substituted or unsubstituted alkyl; alkoxy; or thioalkoxy; $R^1$ and $R^2$ may be taken together with carbon atoms in the phenyl ring to which they are attached to form a cyclic ring; or $R^3$ and $R^4$ may be taken together with the carbon atoms at the 4- and 5-positions of the pyrazolidinone ring to form a cyclic ring; and,

J is a removable blocking group; said compound being stable at a temperature equal to or less than 0°C in the presence of a strong base having a pKa greater than 20; with

B) an electrophilic moiety at a temperature equal to or less than 0°C in the presence of a strong base having a pKa greater than 20; whereby said electrophilic moiety selectively adds to the C atom at the 4-position in the pyrazolidinone ring; and

(2) removing said blocking group.

2. The process of claim 1 characterized in that said J is

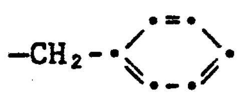

,

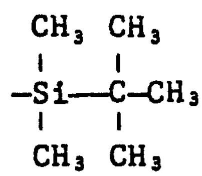

, or

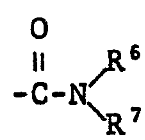

,

wherein:

$R^6$ and $R^7$ are independently methyl, phenyl or

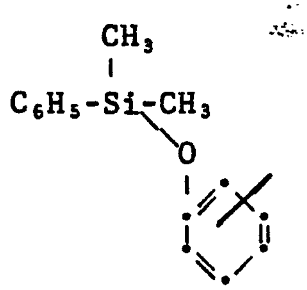

.

3. The process of claim 1or 2 characterized in that said blocking group is removed by hydrolysis or hydrogenolysis.

4. The process of claim 1, 2 or 3 characterized in that said compound is formed by reacting a 3-pyrazolidinone having the structural formula:

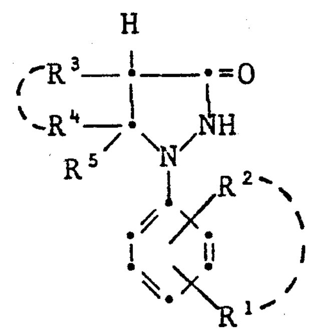

wherein:

$R^1$, $R^2$, $R^3$, $R^4$ and $R^5$ are defined as in claim 1 with a blocking group providing composition which comprises t-butyl-dimethylsilyl chloride; benzyl bromide; or N,N-disubstituted carbamoyl chloride.

5. The process of claim 1, 2 or 3 characterized in that said compound is formed by reacting a 3-chloro-2-pyrazoline having the structural formula:

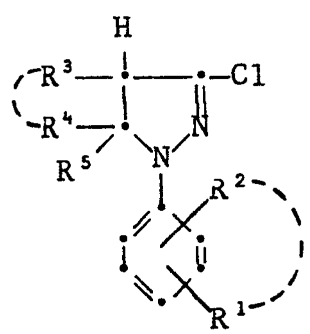

wherein:

$R^1$, $R^2$, $R^3$, $R^4$ and $R^5$ are as defined in claim 1 with a blocking group providing composition comprising potassium benzyloxide.

6. The process of claim 5 characterized in that said 3-chloro-2-pyrazoline is formed by reacting a 3-pyrazolidinone having the structural formula:

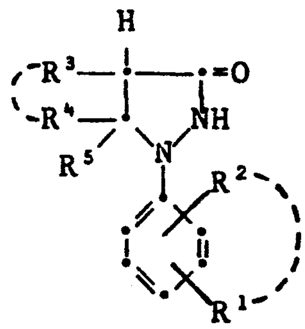

wherein:

$R^1$, $R^2$, $R^3$, $R^4$ and $R^5$ are as defined in claim 1, with an acid chloride.

European Patent Office

**0134696**

Application number

# EUROPEAN SEARCH REPORT

EP 84305247.3

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| D,A | EP - A1 - 0 054 002 (CIBA-GEIGY)<br>* Claims 1,14 *<br>-- | 1 | C 07 D 231/08<br>C 07 D 231/54//<br>G 03 C 5/54 |
| D,A | US - A - 2 289 367 (J.D. KENDALL)<br>* Claim 1 *<br>---- | 1 | |
| | | | **TECHNICAL FIELDS SEARCHED (Int. Cl.4)** |
| | | | C 07 D 231/00 |

The present search report has been drawn up for all claims

| Place of search | Date of completion of the search | Examiner |
|---|---|---|
| VIENNA | 31-10-1984 | BRUS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document